# EUROPEAN PATENT APPLICATION

(11) **EP 4 599 856 A1**
(43) Date of publication of application: **13.08.2025**
(21) Application number: 25179765.0
(22) Date of filing: 18.02.2020
(51) Int. Cl.: A61L 17/00, A61B 17/03, A61B 17/04

(54) **SCAR REDUCING WOUND CLOSURE MATERIALS**

(30) Priority: 18.02.2019 US 201962807184 P
(62) Divisional of application: 20759509.1
(71) Applicant: Lankenau Institute for Medical Research, Wynnewood, PA 19096 (US)
(72) Inventor: HEBER-KATZ, Ellen, 405 S. 13th Street, Philadelphia 19147 (US); PRENDERGAST, George, C., 112 Righters Mill Road, Penn Valley, PA 19072 (US); HOFFMAN, Ryan, 100 E. Lancaster Avenue, Wynnewood, PA 19096 (US); ASLANUKOV, Azamat, Raufavich, 9996 Sandy Road, Philadelphia, PA 19115 (US)
(74) Representative: Appleyard Lees IP LLP

(57) **Abstract**

A composition comprising a wound-closure material physically or chemically associated with an agent that reduces scarring and improves the integrity of skin and underlying tissue in a mammalian subject. Methods for reducing or eliminating scarring or improving mammalian skin integrity comprise closing a wound with a composition, such as a suture material associated with a PHD inhibitor molecule, *e.g.*, 1,4-DPCA.

## Description

### BACKGROUND OF THE INVENTION

Wound closure techniques include a variety of suturing materials such as synthetic sutures, absorbable sutures, natural glues, surgical staples, tapes, and cyanoacrylate tissue adhesives. Although surgical wound closure both speeds and enhances the biological processes of healing by joining the wound edges and minimizing new tissue formation within the wound, even the most exquisitely performed wound closure surgical technique often results in life-long scars. In addition, other problems (e.g., reactivity with suture materials and premature reabsorption) can occur with sutures and lead to an undesirable scarring, which can be both a cosmetic and function problem for the subject with the scarring. Even in cases where cosmetic appearance is not an issue, scarred tissue lacks normal tissue flexibility and is not as strong as normal tissue, sometimes leading to tissue tears when a patient re-bruises the healed injury site.

A scar is a fibrous tissue that replaces normal tissues destroyed by injury or disease. Collagen fibers result in a noticeable scar since normal tissue architecture is not achieved in the wound repair process. After the wound has healed, the scar continues to alter as new collagen is formed and the blood vessels return to normal. While scars can fade over years post-injury, there is some visible evidence of the injury, and hair follicles and sweat glands do not grow back. A cutaneous scar is a dermal fibrous replacement tissue, which results from a wound that had healed by resolution (wound repair) rather than regeneration. Final appearance is influenced largely by the interval between wounding and complete healing 2 to 3 weeks later. Once the scar has formed, it undergoes several distinct macro- and microscopic changes during the maturation process and is completed on average after one year. See, e.g., BOND, J. et al., Maturation of the Human Scar: An Observational Study. 2008, May; Plastic and Reconstructive Surgery, vol. 121 (5): 1650-1658. Patients under 30 years exhibit a slower rate of scar maturation and poorer final appearance than patients over 55 years. The redness of a scar fades after 7 months and, in contrast with rubor (redness) elsewhere, does not reflect an inflammatory process (after the first month). See, e.g., BOND, J. et al., Scar Redness in Humans: How Long Does It Persist after Incisional and Excisional Wounding? 2008, Feb; Plastic and Reconstructive Surgery, 121 (2):487-496. The scar is devoid of dermal appendages and never reaches the same tensile strength as the surrounding skin. See, BEANES, S. et al., Skin repair and scar formation: the central role of TGF-beta. 2003, Mar; Expert Reviews in Molecular Medicine, 5(8):1-22.

Despite advances in wound closure materials and wound repair, there remains no effective medical or surgical approach to prevent scarring associated with fibrotic deposition during wound healing. Wounds, sutured or otherwise closed, leave scars after healing.

### SUMMARY OF THE INVENTION

The present invention is defined in the appended set of claims.

Disclosed is a composition that comprises a wound-closure material physically or chemically associated with an agent that reduces scarring and improves the integrity of skin and underlying tissue in a mammalian subject. The wound-closure material may be a suture; and the agent may be a proline hydroxylase inhibitor compound (PHDi) or prodrug thereof.

Further disclosed is a composition comprising a wound closure material that is dissolvable *in situ* and is physically or chemically associated with an agent that reduces scarring or improves skin integrity, wherein the material releases the agent into the site of the wound as the material dissolves *in situ.*

Further disclosed is a suture material impregnated or coated with, or chemically associated with an agent that reduces scarring and improves the integrity of skin and underlying tissue in a mammalian subject. The agent may be a proline hydroxylase inhibitor compound (PHDi) or prodrug thereof. The compound may be released into the area of the wound during healing as the suture dissolves *in situ.*

Further disclosed is a method for reducing scarring caused by healing of a mammalian wound comprises closing a wound with a wound closure composition as provided herein.

Also disclosed are advantages of these compositions and methods are described further in the following detailed description.

### BRIEF DESCRIPTION OF THE DRAWINGS

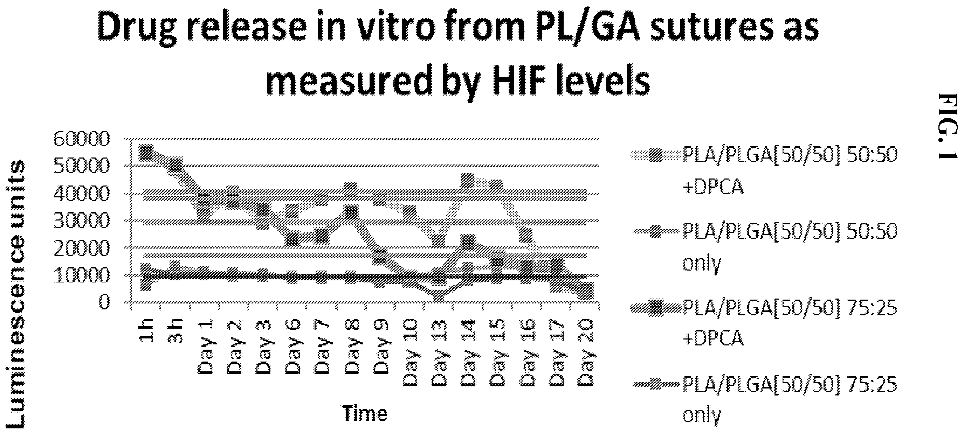

FIG. 1 is a graph plotting drug release *in vitro* from poly(lactic acid)/poly(lactic-co-glycolic acid (PLA/PLGA) sutures over time. Briefly, a mixture of PLA and PLA/GPLA suture was infused with 1,4-DPCA molecules during generation of the sutures. The concentration of DPCA was in all cases 5 mg/100 mg suture material as described below. The sutures, having different ratios of PLA and PLA/PLGA as described below, were tested for rate of drug release. Drug release was measured by HIF levels. The sutures were soaked in medium without serum for 24 hours and transferred to new medium for each timepoint identified on the graph. The medium was tested for HIF1a protein activity in a luciferase assay. The horizontal lines are controls as noted: at about 10000 luminescence units (LU) is a control for DMEM only; at about 18000 LU is a control for 25 µM DCPA; at about 29500 LU is a control for 50 µM DCPA; at about 39000 LU is a control for 100 µM DCPA; and at about 40500 LU is a control for 200 µM DCPA. The curve that is substantially at 10000 LU but dips below at day 12 represents a mixture of PLA and PLA/PLGA [50:50] at a ratio of 75 PLA :25 PLA/PLGA. The curve that starts below 10000LU and increases to about 15000 LU at day 14.5 is PLA and PLA/PLGA [50:50] at a ratio of 75:25 + DPCA. The curve that starts above 50000 and has a peak at day 15 above 40000LU is PLA and PLA/PLGA[50:50] at a ratio of 50:50 + DPCA. The curve that starts above 50000LU and has a peak at day 7.5 at about 34000LU represents PLA and PLA/PLGA [50:50] at a ratio of 75:25 + DPCA.

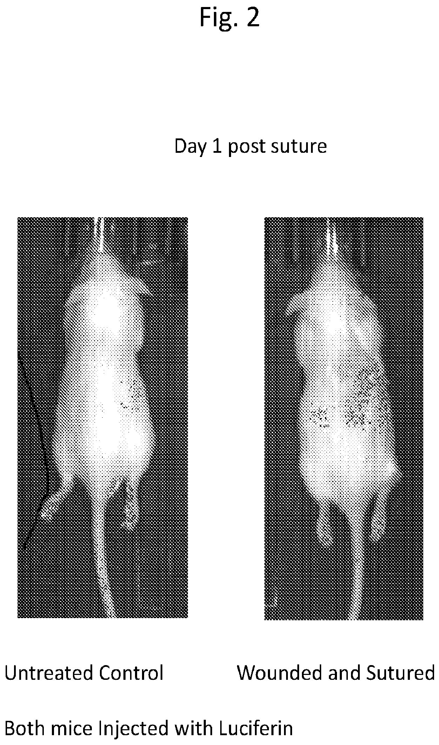

FIG. 2 shows a photograph of an HIF reporter mouse injected with luciferin but otherwise untreated (left) vs. a mouse having a 1 cm linear incision on the right side; the incision was sutured with a suture mixture of PLA and PLA/PLGA[50:50] with DPCA at 5 mg/100 mg suture material sandwiched between two silk sutures (wounded and sutured, right). In the presence of luciferin, the HIF reporter mouse demonstrates bioluminescence where HIF is produced. The increase in bioluminescence at Day 1 post-suture indicates that the drug was being detected at the point of the suture, as evidenced by an increase in HIF detected by luciferin.
FIG. 3 shows a photograph two similar mice as in FIG. 2 but at Day 3 post suture. There is again additional increase in bioluminescence at the site of the wound.
FIG. 4 shows a photograph two similar mice as in FIG. 2 but at Day 7 post suture. There is again additional increase in bioluminescence at the site of the wound.

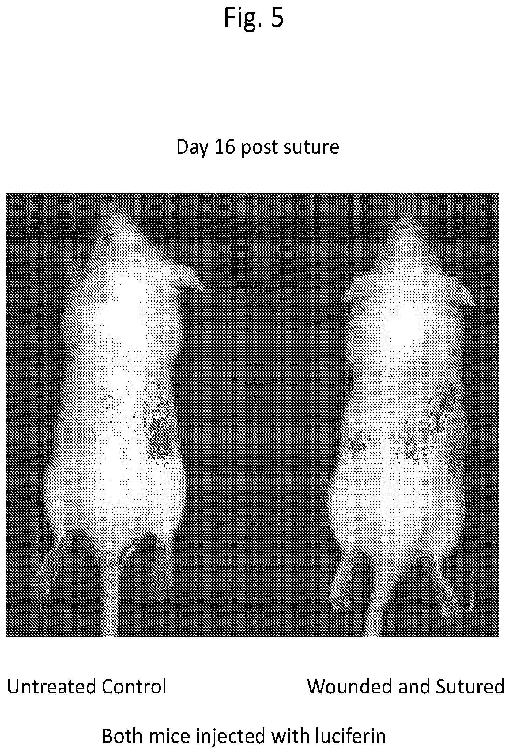

FIG. 5 shows a photograph two similar mice as in FIG. 2 but at Day 15 post suture. By Day 15 the bioluminescence is reduced which demonstrates that release of the drug from the suture *in vivo* is similar to release *in vitro* in FIG. 1.
FIG. 6 is a picture of a 1 cm linear incision on the right side of mouse skin; the incision was sutured with a suture mixture of PLA and PLA/PLGA at a ratio of 50:50 with DPCA at a concentration of 5 mg/100 mg suture sandwiched between two silk sutures.
FIG. 7 shows two pictures of the normal epidermis of an HIF reporter mouse, trichrome stained, with the darker (blue) epidermis indicating the level of collagen.
FIG. 8 shows the histological analysis of the wound area around the treated suture on Day 22 post injury. The trichrome-stained skin of the mouse of FIG. 7 has a suture of FIG. 6 at point A. The area around the suture shows reduced blue color at point B, indicating reduced collagen (i.e., a potential reduction in fibrosis), and increased epidermal thickness at point C, which leads to enhanced healing.

### DETAILED DESCRIPTION

Scarless healing offers a widely desired feature to wound closure/wound healing products generally, including sutures. As described herein, the inventors have provided wound closure materials and methods of use that substantially reduce the scarring that typically results after wound closure, particularly sutured wound closure. These compositions and methods are designed to solve or reduce the problem of scarring during healing after surgery. In one disclosure, the compositions and methods described herein use reduced scaring (through lack of collagen crosslinking) as well as epimorphic regeneration and agents that can engender reduced scaring and epimorphic regeneration as a component of a wound closure material, such as a suture to reduce the scarring that typically results from sutured or non-sutured wound healing.

### Components and Definitions of the Compositions and Methods

In this specification, technical and scientific terms used herein have the same meaning as commonly understood by one of ordinary skill in the art to which this invention belongs and by reference to published texts, which provide one skilled in the art with a general guide to many of the terms used in the present application. The definitions contained in this specification are provided for clarity in describing the components and compositions herein.

By "subject" or "patient" as used herein is meant a male or female mammalian animal, including a human, a domestic animal or pet, animals normally used for clinical research. Still other mammalian animals that can benefit from treatment may include animals of high value, such as horses and zoo animals. In one disclosure, the subject of these methods and compositions is a human. Still other suitable subjects include, without limitation, murine, rat, canine, feline, porcine, bovine, ovine, equine, and others.

By "wound" as used herein is meant wherein the wound is an incisional wound, a laceration, an abrasion, a puncture, a traumatic wound, a diabetic wound, a pressure wound, a burn wound, a chronic wound, or a combination thereof.

As used herein the term "wound closure material" refers to sutures, surgical staples, and wound closure adhesives, among other products suitable for wound closure during healing.

"Sutures" includes natural and synthetic materials, absorbable and nonabsorbable materials, and monofilament and multifilament materials. Examples of natural materials include gut, silk, and even cotton. Gut is considered an absorbable monofilament, whereas silk and cotton are braided non-absorbable multifilament. Various absorbable and nonabsorbable synthetic materials are available for suturing. Absorbable sutures are useful in repair of a wound that heals quickly and needs minimal temporary support and are used for alleviating tension on wound edges. Examples of absorbable sutures include the monofilamentous Monocryl^{®} sutures (poliglecaprone), Maxon^{®} sutures (polyglycolide-trimethylene carbonate), and PDS^{®} sutures (polydioxanone). Braided absorbable sutures include Vicryl^{®} sutures (polyglactin), Dexon^{®} sutures (polyglycolic acid), and cat gut. Nonabsorbable sutures offer longer mechanical support, compared to absorbable suture materials, which lose their tensile strength before complete absorption. Gut can last 4-5 days in terms of tensile strength. In the chromic form (*i.e.,* treated in chromic acid salts), gut can last up to 3 weeks. Vicryl^{®} sutures and Dexon^{®} sutures maintain tensile strength for 7-14 days, although complete absorption takes several months. Polytrimethylene Carbonate Sutures (Maxon^{®} sutures) and Polydioxanone (PDS^{®} sutures) are longer-term absorbable sutures, lasting several weeks and requiring several months for complete absorption. Nonabsorbable sutures have varying tensile strengths and may be subject to some degree of degradation. Nonabsorbable sutures include silk, nylon, Prolene^{®} sutures (polypropylene), Novafil^{®} sutures (polybutester), polytetrafluoroethylene (PTFE) sutures and polyester sutures.

Surgical staples are composed of stainless steel, which has been shown to be less reactive than traditional suturing material. The act of stapling requires minimal skin penetration, and, thus, fewer microorganisms are carried into the lower skin layers. Staples are expensive and require great care in placement, especially in ensuring the eversion of wound edges.

Surgical adhesives also facilitate wound closure. One substance, cyanoacrylate, easily forms a strong flexible bond. The cyanoacrylate tissue adhesives are liquid monomers that polymerize on contact with tissue surfaces in an exothermic reaction creating a strong yet flexible film that bonds the apposed wound edges. Octyl-2-cyanoacrylate (Dermabond^{®}, Ethicon, Somerville, N.J.) is the only cyanoacrylate tissue adhesive approved by the U.S. Food and Drug Administration (FDA) for superficial skin closure. Octyl-2-cyanoacrylate is not for use in subcutaneous wounds. Subcutaneous sutures are used to take the tension off the skin edges prior to applying the octyl-2-cyanoacrylate. Subcutaneous suture placement aids in averting the skin edges and minimizing the chances of deposition of cyanoacrylate into the subcutaneous tissues.

Fibrin-based tissue adhesives are generated from autologous sources or pooled blood and can seal tissues. Although they do not have adequate tensile strength to close skin, fibrin tissue adhesives are useful to fixate skin grafts or seal cerebrospinal fluid leaks. FDA-approved fibrin tissue adhesives made from pooled blood sources include the commercial preparations Tisseel^{®} adhesive (Baxter) and Hemaseel^{®} adhesive (Haemacure). These fibrin tissue adhesives are relatively strong and can be used to fixate tissues. Autologous forms of fibrin tissue adhesives can be made from patient's plasma.

Adhesive tapes or strips allow wound edges to be joined and splinted. Porous paper tapes (*e.g.*, Steri-Strips^{®} tape) ensure proper wound apposition and provide additional suture reinforcement. These tapes can be used either with sutures or alone. Often, skin adhesives (*e.g.*, Mastisol^{®} adhesive, tincture of Benzoin) aid in tape adherence. The ClozeX^{®} adhesive strip (Wellesley, Mass.) permits rapid and effective wound closure at a significantly lower cost than suturing or using a tissue adhesive. Another adhesive tape is Embrace^{®} tape, a silicone tape to assist wound closure. However, adhesive strips are not appropriate for many types of lacerations.

"Epimorphic regeneration (ER)" is a healing process in which appendages and organs can regenerate to normal structure and function in the absence of scar in a manner similar to an embryonic-type healing. The inventors discovered that transient induction of the molecule HIF-1a, a master regulator of tissue responses to hypoxia and other tissue stresses, is essential to enable ER. See, e.g., US Patent No. 9675607; HEBER-KATZ, E and Messersmith, P, Drug Delivery and Epimorphic Salamander-type Mouse Regeneration: A Full Parts and Labor Plan. 2018 Mar; Adv. Drug Delivery Rev., 129:254-261; HEBER-KATZ, E., Oxygen, Metabolism, and Regeneration: Lessons from Mice, Trends in Mol. Med., 2017 Nov; 23(11):1024-1036; and ZHANG, Y et al., Drug-induced regeneration in adult mice, Sci Transl Med. 2015 June; 7 (290): 290ra92. The expression of HIF-1a protein in mammals is known to be regulated by proline hydroxylase enzymes (PHDs) also known as EGLNs.

By the phrase "an agent that reduces scarring and improves the integrity of skin and underlying tissue in a mammalian subject" includes small molecules, peptides, proteins, DNA and RNA sequences that demonstrate certain functional activities. Among such agents are those that increase or stabilize hypoxia-inducible factor 1 (HIF1 or HIF1-alpha), such as PHD inhibitors discussed below. Other suitable agents that inhibit or promote protein in the HIF regulatory pathway can lead to transient HIF upregulation or stabilization. See, e.g., MASOUD, GN and LI, W. HIF-1α pathway: role, regulation and intervention for cancer therapy. 2015; Sept; Acta Pharmaceutica Sinica B, 5(5):3780-389,

Still other suitable agents are those that decrease expression of the cyclin-dependent kinase inhibitor p21 or inhibit p21. See, e.g., ABBAS, T and Dutta, A, P21 in cancer: intricate networks and multiple activities, Nat Rev Cancer, 2009 Jun; 9(6):400-414; and BADALBAEVA, K et al. Lack of p21 expression links cell cycle control and appendage regeneration in mice. 2010, Mar; Proc Natl Acad Sci, USA, 107: 5845.

Some inhibitors of p21 include butyrolactoneI, sorafenib, UC2288, LLW10, Daprodustat (GSK1278863), Vadadustat (AKB-6548), Molidustat (Bay 85-3934), Roxadustat (FG-4592), Desidustat (also known as ZYAN1) and siRNA to p21 and Mir 17-92. See, *e.g.,* LIU, R et al, Small-molecule inhibitors of p21 as novel therapeutics for chemotherapy-resistant kidney cancer. Future Med Chem, 2013 Jun; 5(9):991-994; DIB, J, et al. Mass spectrometric characterization of the hypoxia-inducible factor (HIF) stabilizer drug candidate BAY 85-3934 (molidustat) and its glucuronidated metabolite BAY-348, and their implementation into routine doping controls. 2010, Jan; Drug Testing and Analysis 9(1): 61-67; EICHNER, D, et al. Implementation of the prolyl hydroxylase inhibitor Roxadustat (FG-4592) and its main metabolites into routine doping controls. 2017 Nov; Drug Testing and Analysis. 9 (11-12): 1768-1778 (epub.2017 May); JAIN, M., et al. Pharmacological Characterization of ZYAN1, a Novel Prolyl Hydroxylase Inhibitor for the Treatment of Anemia. 2015, Sept; Drug Research. 66 (02): 107-112; THEVIS M, et al. Mass spectrometric characterization of a prolyl hydroxylase inhibitor GSK1278863, its bishydroxylated metabolite, and its implementation into routine doping controls. 2016 Aug; Drug Test Anal. 8(8):858-63; and MARTIN ER, et al. Clinical Trial of Vadadustat in Patients with Anemia Secondary to Stage 3 or 4 Chronic Kidney Disease. 2017, Mar.; Am J Nephrol. 45(5):380-388.

By "prolyl hydroxylase domain enzymes" or "PHDs" is meant a family of enzymes that catalyzes the hydroxylation of certain prolyl residues in collagen precursors using molecular oxygen, ferrous ion, ascorbic acid, and α-ketoglutarate. The members of this family include PHD1, PHD2, and PHD3. They are non-heme iron containing dioxygenases that require oxygen and 2-oxoglutarate as co-substrates and iron and ascorbate as cofactors for their enzymatic activity. See, *e*.*g*., GUPTA N and Wish JB, Hypoxia-Inducible Factor Prolyl Hydroxylase Inhibitors: A Potential New Treatment for Anemia in Patients With CKD. 2017 June; Am. J. Kidney Dis., 69(6):815-826 (epub Feb 2017). One form of a PHD2 enzyme (prolyl 4-hydroxylase) synthesizes 4-hydroxyprolyl residues, while another produces 3-hydroxyprolyl residues. Among PHDs are two long-known collagen prolyl-4-hydroxylases (MYLLYHARJU J, Prolyl 4-hydroxylases, the key enzymes of collagen biosynthesis, 2003 Mar; Matrix Biol. 22(1):15-24), the more recently identified FIH-1 (factor inhibiting HIF), and PHD1-3, asparaginyl and prolyl hydroxylases, responsible for HIF-1α protein hydroxylation.

Certain small molecules useful as agents in the compositions described herein are the "prolyl hydroxylase domain inhibitors" or "PHDi" small molecules that inhibit or stabilize HIF. Among these are Roxadustat (FG-4592; Fibrogen) described in EICHNER, D *et al,* cited above. Another molecule is Vadadustat (AKB-6548; Akebia) described in MARTIN ER *et al,* cited above. Another PHD inhibitor is Daprodustat (GSK-1278863; GlaxoSmithKline) and Molidustat (BAY 85-3934; Bayer). These four above-mentioned PHD inhibitors are described and their dosages in clinical trials detailed in GUPTA and Wish JB, 2017, cited above. Still other PHD inhibitors useful in the methods described herein are a prodrug of 1,4-DPCA or 1,4-dihydrophenonthrolin-4-one-3-carboxylic acid, or a salt of 1,4-DPCA, Imiquimod or CoCl₂ described in US patent application publication No. 20150320877, published Nov. 12, 2015. Still other small molecule PHD inhibitors include dimethyloxalylglycine (DMOG; CAS 89464-63-1) and desferrioxamine B, also known as 30-amino-3,14,25-trihydroxy-3,9,14,20, 25-pentaazatriacontane-2,10,13,21,24-pentone, or a salt thereof; CAS 70-51-9 (EDELMAYER, M et al, Effect of prolyl hydroxylase inhibitor-loaded collagen barrier membranes on osteoclastogenesis and osteoblastogenesis. 2017 May; J. Biomater. Appl., 31(10):1370-1379) and ethyl-3,4-dihydroxybenzoate (EDHB) (HARNOSS JM et al, Prolyl Hydroxylase Inhibition Enhances Liver Regeneration Without Induction of Tumor Growth. 2017 Apr.; Ann Surg., 265(4):782-791. Additional inhibitors that are described in the art include Nepicastat (SYN-117) HCl, (R)-Nepicastat HCl Tetrahydropapaverine HCl, and Norlaudanosine H. See, also, MAXWELL PH and Eckardt KU, HIF prolyl hydroxylase inhibitors for the treatment of renal anaemia and beyond. 2016 Mar; Nat. Rev. Nephrol. 12(3):157-168.

Such publicly described PHD inhibitor compounds and molecules and their salts derived from pharmaceutically or physiologically acceptable acids, bases, alkali metals and alkaline earth metals are useful in the methods described herein. Still other PHD inhibitors may be found in the catalogs of various biochemical and pharmaceutical suppliers.

Still another suitable small molecule agent is Ciclopirox, a molecule having the formula C₁₂H₁₇NO₂, PubChem CID 2749. This agent is a synthetic, broad-spectrum antifungal agent with antibacterial and anti-inflammatory activities. Yet another suitable agent is dibenzoylmethane, or 1,3-Diphenyl-1,3-propanedione, having the formula C₁₅H₁₂O₂, PubChem CID 8433, which is a beta-diketone and an aromatic ketone known to exhibit antimutagenic and anticancer effects. It has a role as an antineoplastic agent, a metabolite and an antimutagen. It is available from public sources, *e*.*g*., Sigma-Aldrich. Yet another suitable small molecule for use in the compositions described herein is Deferoxamine (DFO) having the formula C₂₅H₄₈N₆O₈, PubChem CID No. 2973. Deferoxamine is an iron-chelating agent that binds free iron in a stable complex, preventing it from engaging in chemical reactions. Deferoxamine chelates iron from intra-lysosomal ferritin and siderin forming ferrioxamine, a water-soluble chelate excreted by the kidneys and in the feces via the bile. This agent does not readily bind iron from transferrin, hemoglobin, myoglobin or cytochrome. (NCI04). Still another suitable small molecule for use in these compositions is hydralazine (also 1-Hydrazinophthalazine) which has the formula C₈H₈N₄ and is a phthalazine derivative with antihypertensive effects. It is available as a variety of salts from public pharmaceutical sources. Still other small molecules are useful.

Physiologically acceptable acids of the small molecule compounds identified above include those derived from inorganic and organic acids. A number of inorganic acids are known in the art and include, without limitation, hydrochloric, hydrobromic, hydroiodic, sulfuric, nitric, and phosphoric acid. A number of organic acids are also known in the art and include, without limitation, lactic, formic, acetic, fumaric, citric, propionic, oxalic, succinic, glycolic, glucuronic, maleic, furoic, glutamic, benzoic, anthranilic, salicylic, tartaric, malonic, mallic, phenylacetic, mandelic, embonic, methanesulfonic, ethanesulfonic, panthenoic, benzenesulfonic, toluenesulfonic, stearic, sulfanilic, alginic, and galacturonic acids. Inhibitor compound salts can be also in the form of esters, carbamates, sulfates, ethers, oximes, carbonates, and other conventional "pro-drug" forms, which, when administered in such form, convert to the active moiety *in vivo.* In one disclosure, the prodrugs are esters. The compounds discussed herein also encompass "metabolites" which are unique products formed by processing the selected inhibitor compound by the cell or subject. In one disclosure, metabolites are formed *in vivo.*

Also useful as agent that reduce scarring and improves the integrity of skin and underlying tissue in a mammalian subject by association with a wound closure material described herein are "antisense" nucleotide sequence or a small nucleic acid molecule having a complementarity to the nucleic acid sequence of a selected PHD or p21 target described above. Such an antisense sequence can also function as a PHD inhibitor or p21 inhibitor in the methods described herein, such as a nucleic acid sequence having complementarity to a sense region of the small nucleic acid molecule. For example, in one disclosure, the composition comprises a nucleic acid construct comprising a sequence that reduces or suppresses the expression of one of the PHD enzymes, p21 targets or a combination thereof. Additionally, or alternatively, in one disclosure, the composition comprises a PHD-inhibitory short nucleic acid molecule (e.g., siRNA). In one disclosure, the PHD-inhibitory short nucleic acid molecule blocks the PHD2 pathway. Such agents that create a PHD blockade lead to p21 downregulation in a manner that is critical for epimorphic regeneration.

In a further disclosure, the PHD-inhibitory short nucleic acid molecule transiently upregulates HIF1 or other molecules involved in unleashing the latent potential for ER in mammals. For example, the inhibiting composition can include a nucleic acid construct comprising a short nucleic acid molecule selected from the group consisting of a short hairpin RNA (shRNA), a short interfering RNA (siRNA), a double stranded RNA (dsRNA), a micro RNA, and an interfering DNA (DNAi) molecule, optionally under the control of a suitable regulatory sequence

Still other agents that reduce scarring and improves the integrity of skin and underlying tissue in a mammalian subject when in association with a wound closure material described herein are certain peptides and/or proteins. Such proteins can be antibodies (or antibody fragments) that can bind and thus inhibit the activity of PHD or p21 enzymes or proteins in their respective pathways. In one disclosure, p21 agonists align downstream or in parallel with the PHD pathway which limits ER and hence is unleashed by PHDi or PHD siRNA.

In one disclosure, the additional peptide agents useful in these wound closure compositions are HIF modulators / ER agonists, such as secreted frizzled related protein 2 (sFRP2) and protease-activated receptor 1 (PAR 1) agonists. sFRP2 is expressed both in the epidermis and dermis of human normal skin. This peptide has been shown to enhance regeneration (data not shown). Skin cells (melanocytes, keratinocytes, and fibroblasts) express sFRP2 constitutively. KIM, M et al, Secreted Frizzled-Related Protein 2 (sFRP2) Functions as a Melanogenic Stimulator; the Role of sFRP2 in UV-Induced Hyperpigmentary Disorders., 2016, Jan; J. Invest. Dermatol., 136(1):236-244. In one disclosure, the agent that reduces scarring and improves the integrity of skin and underlying tissue includes small molecules, peptides, proteins, DNA and RNA sequences that interfere with the sFRP2 pathway and result in increased expression or activity of sFRP2. In another disclosure, peptides or proteins that are PAR1 agonists or agonists of other members of the sFRP2 pathway or the PAR1 pathway and their many components which lead to PHD regulation, can also be used to promote ER. PAR1 is a prototype member of an established protease-activated receptor family, which has activity in thrombosis, hemostasis, vascular biology and tumor biology. It is upregulated in regenerating mice, is upstream from HIF, and can activate the HIF pathway. A PAR1 agonist is the peptide TRLLRNPNDK SEQ ID NO: 1 and/or the protein thrombin. See, *e.g.,* AUSTIN, KM et al, Matrix metalloproteases and PAR1 activation, Blood, 2013 Jan, 121(3):431-439. In one disclosure, the agent that reduces scarring and improves the integrity of skin and underlying tissue includes small molecules, peptides, proteins, DNA and RNA sequences that interference the PAR1 pathway and result in increased expression or activity of PAR1. These above-identified agents reduce scarring and improves the integrity of skin and underlying tissue in a mammalian subject when in association with a wound closure material, such as above.

The term "in association with" as referred to herein means that the selected agent is physically or chemically associated with the wound closure material. In one disclosure, the physical association means that the agent is formulated to physically coat the surface of the selected wound closure material. In another disclosure, the selected agent may be chemically linked covalently or non-covalently with reactive chemical groups on the surface of the wound closure materials. In one disclosure, the chemical groups can be reaction carboxylic acids, hydroxides or amines that are on the surface of materials, such as catgut sutures, or other synthetic materials. In yet another method of association useful herein is that the selected agent is formulated or admixed with the materials that are used to generate the wound closure materials. For example, such agents can be included in the polymers such as polylactide (PLA) or polyglycolic acid or other glycolide polymers (GPLA) used to generate sutures. Still other means of associating the agents with the selected wound closure materials may be employed in generating the compositions described herein.

For example, a formulation of one of these agents in a pharmaceutically acceptable carrier or excipient can be applied to the surface of the wound closure material and permitted to dry thereon. Such a formulation can include an effective amount of the agent admixed with a solid and/or liquid carrier, in dry, liquid, or semi-solid form which is pharmaceutically acceptable. The compositions are typically sterile solutions or suspensions. Examples of excipients which may be combined with the small molecule agents, peptide agents and/or DNA/RNA agents include, without limitation, solid carriers, liquid carriers, adjuvants, amino acids (glycine, glutamine, asparagine, arginine, lysine), antioxidants (ascorbic acid, sodium sulfite or sodium hydrogen-sulfite), binders (gum tragacanth, acacia, starch, gelatin, polyglycolic acid, polylactic acid, poly-d,l-lactide/glycolide, polyoxaethylene, polyoxapropylene, polyacrylamides, polymaleic acid, polymaleic esters, polymaleic amides, polyacrylic acid, polyacrylic esters, polyvinylalcohols, polyvinylesters, polyvinylethers, polyvinylimidazole, polyvinylpyrrolidon, or chitosan), buffers (borate, bicarbonate, Tris-HCl, citrates, phosphates or other organic acids), bulking agents (mannitol or glycine), carbohydrates (such as glucose, mannose, or dextrins), clarifiers, coatings (gelatin, wax, shellac, sugar or other biological degradable polymers), coloring agents, complexing agents (caffeine, polyvinylpyrrolidone, β-cyclodextrin or hydroxypropyl-β-cyclodextrin), compression aids, diluents, disintegrants, dyes, emulsifiers, emollients, encapsulating materials, fillers, flavoring agents (peppermint or oil of wintergreen or fruit flavor), glidants, granulating agents, lubricants, metal chelators (ethylenediamine tetraacetic acid (EDTA)), osmo-regulators, pH adjustors, preservatives (benzalkonium chloride, benzoic acid, salicylic acid, thimerosal, phenethyl alcohol, methylparaben, propylparaben, chlorhexidine, sorbic acid, hydrogen peroxide, chlorobutanol, phenol or thimerosal), solubilizers, sorbents, stabilizers, sterilizer, suspending agent, sweeteners (mannitol, sorbitol, sucrose, glucose, mannose, dextrins, lactose or aspartame), surfactants, syrup, thickening agents, tonicity enhancing agents (sodium or potassium chloride) or viscosity regulators. See, the excipients in "Handbook of Pharmaceutical Excipients", 5th Edition, Eds.: Rowe, Sheskey, and Owen, APhA Publications (Washington, DC), 2005 and US Patent No. 7,078,053.The selection of the excipient is dependent on the nature of the compound selected and the form of administration desired.

Solid carriers include, without limitation, starch, lactose, dicalcium phosphate, microcrystalline cellulose, sucrose and kaolin, calcium carbonate, sodium carbonate, bicarbonate, lactose, calcium phosphate, gelatin, magnesium stearate, stearic acid, or talc. Fluid carriers without limitation, water, e.g., sterile water, Ringer's solution, isotonic sodium chloride solution, neutral buffered saline, saline mixed with serum albumin, organic solvents (such as ethanol, glycerol, propylene glycol, liquid polyethylene glycol, dimethylsulfoxide (DMSO)), oils (vegetable oils such as fractionated coconut oil, arachis oil, corn oil, peanut oil, and sesame oil; oily esters such as ethyl oleate and isopropyl myristate; and any bland fixed oil including synthetic mono- or diglycerides), fats, fatty acids (include, without limitation, oleic acid), cellulose derivatives such as sodium carboxymethyl cellulose, and/or surfactants.

The agents described above can be optionally co-formulated with other "anti-scarring agents" and also associated with the wound closure materials. Such anti-scarring agents include EXC001 (an anti-sense RNA against Connective Tissue Growth Factor (CTGF); Excaliard Pharmaceuticals), AZX100 (a phosphopeptide analog of Heat Shock Protein 20 (HSP20); Capstone Therapeutics Corp), PRM-151 (recombinant human serum amyloid P/Pentaxin 2; Promedior), PXL01 (a synthetic peptide derived from human Lactoferrin; PharaSurgics AB), DSC127 (an angiotensin analog; Derma Sciences, Inc), RXI-109 (a self-delivering RNAi compound that targets Connective Tissue Growth Factor (CTGF); Galena Biopharma), TCA (trichloroacetic acid; Isfahan University of Medical Sciences), Botox^{®} (Capital District Health Authority and Allergan); Botulium toxin type A (Chang Gung Memorial Hospital), 5-fluorouracil, bleomycin, onion extract, pentoxifylline, verapamil, tacrolimus, and anti-TNFα agents, tamoxifen, tretinoin, colchicine, calcium antagonists, tranilast, zinc, and vitamin E. See publications such as MARSHALL, CD et al, Cutaneous Scarring: Basic Science, Current Treatments and Future Directions, Advances in Wound Care, 2018 Feb; 7(2):29-45; KOC, E et al., An Open, Randomized, Controlled, Comparative Study of the Combined Effect of Intralesional Triamcinolone Acetonide and Onion Extract Gel and Intralesional Triamcinolone Acetonide Alone in the Treatment of Hypertrophic Scars and Keloids, 2008, Nov; Dermatologic Surg, 34(11): 1507-1514 and other publications).

The term "reduce" or "eliminate" or variations thereof as used herein refers to the ability of the compositions described herein to reduce the occurrence and appearance of scarring following a wound closure or wound healing in the subject as compared to other known scar reduction techniques. In one disclosure, the term "reduce" or "eliminate" or variations thereof as used herein refers to an about 5%, about 10%, about 20%, about 30%, about 40%, about 50%, about 60%, about 70%, about 80%, about 90%, about 95%, about 97%, about 99% of the reduction as compared to other known scar reduction techniques.

The term "increase" or "promote" or variations thereof as used herein refers to a change to about 105%, about 110%, about 120%, about 130%, about 140%, about 150%, about 160%, about 170%, about 180%, about 190%, about 195%, about 197%, about 199%, about 2 folds, about 4 folds, about 5 folds, about 10 folds or more of the reference or control.

When in association with the wound closure materials/wound healing materials described above, the agents are incorporated in amounts effective to achieve the scar reduction/elimination and improvement of skin integrity. By "effective amount" of these agents when in association with the wound healing materials/wound closure materials means the amount or concentration present in a wound closure material, which is released into the site of the wound sufficient to retard, suppress, reverse or inhibit scarring, while providing the least negative side effects to the treated subject. In one disclosure, in which the suture or other wound closure material is in contact with subdermal layers of skin, the agent is administered subdermally. In one disclosure, such a dose released by the suture is similar to that released by a subdermal pellet. In another disclosure, the agent is administered to other layers of the skin, e.g., to the epidermis.

In one disclosure, the effective dose of agent (small molecule/peptide/nucleic acid) released from the wound closure material is at least 0.1, 0.2, 0.3, 0.4, 0.5, 0.6, 0.7, 0.8, 0.9, 1, 2, 3, 4, 5, 6, 7, 8, 9, or at least 10 or more mg per over a suitable time period.

In one disclosure, the effective dose of agent is expressed as drug loaded per inch of suture material, i.e., about 0.01 mg per inch of suture material to about 1 mg per inch of suture material which is released to the wound in situ over a suitable time period. In another disclosure, the effective dose or load of agent is about 0.01 mg, about 0.02, about 0.03, about 0.04, about 0.05, about 0.06, about 0.07, about 0.08, about 0.09, about 0.10, about 0.11, about 0.12, about 0.13, about 0.14, about 0.15, about 0.16, about 0.17, about 0.18 about 0.19, about 0.20, about 0.21, about 0.22, about 0.23, about 0.24, about 0.25, about 0.26, about 0.27, about 0.28 about 0.29, about 0.30, about 0.31, about 0.32, about 0.33, about 0.34, about 0.35, about 0.36, about 0.37, about 0.38 about 0.39, about 0.40, about 0.41, about 0.42, about 0.43, about 0.44, about 0.45, about 0.46, about 0.47, about 0.48 about 0.49, about 0.50, about 0.51, about 0.52, about 0.53, about 0.54, about 0.55, about 0.56, about 0.57, about 0.58 about 0.59, about 0.60, about 0.61, about 0.62, about 0.63, about 0.64, about 0.65, about 0.66, about 0.67, about 0.68 about 0.69, about 0.70, about 0.71, about 0.72, about 0.73, about 0.74, about 0.75, about 0.76, about 0.77, about 0.78 about 0.79, about 0.80, about 0.81, about 0.82, about 0.83, about 0.84, about 0.85, about 0.86, about 0.87, about 0.88 about 0.89, about 0.90, about 0.91, about 0.92, about 0.93, about 0.94, about 0.95, about 0.96, about 0.97, about 0.98 about 0.99, about 1.00 mg per inch of suture material for release over a suitable time period. In a further disclosure, the effective dose of agent is about 0.1 mg per inch of suture material over a suitable time period. In one disclosure, the suitable time period is about 1 to 35 days, including each and every integer within the range. In a further disclosure, the time period is about 1 day to about 14 days. In yet another disclosure, the time period is about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, about 20 days, about 21 days, about 4 weeks or about 5 weeks.

In another disclosure, the effective amount of the peptide released from the wound closure/healing composition is within the range of 1 mg/kg body weight to 100 mg/kg body weight in humans including all integers or fractional amounts within the range. In certain disclosures, the effective amount is at least 2, 5, 10, 15, 20, 25, 30, 35, 40, 45, 50, 55, 60, 65, 70, 75, 80, 85, 90, 95 or 100 mg/kg body weight, including all integers or fractional amounts within the range. In one disclosure, the above amounts define an amount delivered to the subject per day. In still other disclosures, these amounts represent the amount delivered to the subject over more than a single day.

The terms "a" or "an" refers to one or more. For example, "an expression cassette" is understood to represent one or more such cassettes. As such, the terms "a" (or "an"), "one or more," and "at least one" are used interchangeably herein.

As used herein, the term "about" means a variability of plus or minus 10 % from the reference given, unless otherwise specified.

The words "comprise", "comprises", and "comprising" are to be interpreted inclusively rather than exclusively, *i*.*e*., to include other unspecified components or process steps. The words "consist", "consisting", and its variants, are to be interpreted exclusively, rather than inclusively, *i.e.,* to exclude components or steps not specifically recited.

### Compositions and Methods for Reducing Scarring and Enhances Skin Integrity

In one disclosure, therefore, a composition comprises a wound closure material physically or chemically associated with an agent that reduces scarring and improves the integrity of skin and underlying tissue in a mammalian subject. Any of the agents described above can be used in this composition, including, without limitation, those that transiently increase HIF, decrease p21, interfere with the HIF regulatory pathway leading to HIF upregulation, act as sFRP2 or as PAR1 agonists, and/or target PHD signaling.

As described herein, one such composition is a suture which is associated with one or more of these agents. For example, one disclosure of such a composition is a catgut suture which is associated with a PHD inhibitor small molecule such as 1,4-DPCA. The PHD-inhibiting compound 1,4-DPCA can enable epimorphic regeneration of tissues when incorporated into a slow-release subcutaneous hydrogel (ZHANG *et al.,* cited above). The typical delivery period in this system is over a 2-week period. Accordingly, the drug is delivered gradually leading to systemic release and function, possibly in a specific way to macrophages or other phagocytosing cells that degrade the hydrogel and are involved in wound healing.

As described herein, the compound (1,4-DPCA) or any other of the related agents described herein is directly coupled to a suture material that dissolves over a similar 2-week period, thereby releasing the compound over this same period of time to enable a reduction in scarring and even possibly scarless healing after surgery. Since suture interacts directly with the skin and underlying subdermal tissues, this modified suture can lead to a nonscarring healing (regenerative) response. Such sutures are useful for facial skin, plastic surgery, or skin anywhere on the body where scars after surgery are undesired.

Cat gut (protein) sutures used specifically for facial stitches degrade after 2 weeks, which is a useful release time for 1,4-DPCA. Multiple amino acid side chains for coupling exist on the cat gut sutures, and as the suture degrades, the drug is released. Cat gut is made of extracellular matrix components with the majority being collagen. The more collagen, the better the suture. The compound 1,4-DPCA inhibits P4H, a collagen specific prolyl-hydroxylase. Thus, by adding 1,4-DPCA to the gut suture, any induced P4H is blocked and leads to reduced scar formation by reducing crosslinking of the wound-induced extracellular matrix such as collagen. While not wishing to be bound by theory, the inventors believe that the level of collagen release from the gut suture activates the enzyme P4H or prolyl hydroxylase which acts specifically on 30% of the prolines in collagen to allow the formation of the collagen tertiary structure through crosslinking.

In one disclosure, the agent is coated onto an existing suture material using a suitable formulation. In another disclosure, the selected agent may be chemically coupled via the reactive groups from catgut sutures and/or selected suture materials. For catgut or silk sutures, chemicals that crosslink the selected agent, e.g., the PHDi, to the polymeric backbone of the sutures, e.g., collagen or other extracellular matrix (ECM)-based backbones, can be used. Typical cross-linkers include imidazoles or other standard protein crosslinking agents that can be controlled (e.g. for crosslinking to lysines and prolines). In still another disclosure, the agent may be added to monomeric fluid from which suture material is formed via polymerization reaction. The polymerization reaction thereby cages the agent in the polymeric fiber that is spun out from the monomeric fluid. In one disclosure, 1,4-DPCA is added into mixtures of glycolic and lactic acid and the sutures spooled from this mixture would have embedded agent within them.

Where the materials are biodegradable *in situ,* the sutures would deliver the agents over time as they degraded. Where the sutures or other materials were non-degradable, they would still provide contact between the wound surface and the agent within or coated on the material.

In still another disclosure, a staple of surgical steel can be coated with a formulation containing one or more of the agents described herein allowing contact between the agents and the wound while the staple is in place.

Another disclosure includes coating a wound closure material such as an adhesive or silicone tape such as EMBRACE^{™} material, impregnated or coated with an agent, or adding the agent into the compounds used to generate the adhesive or silicone. Similarly, wound closure materials, sutures or stapes, may be coated with, or prepared with admixtures of the therapeutic agents described herein. Addition of 1,4-DPCA to silicone tapes that assist wound closure (*e*.*g*. Embrace allow the agents to be released to the healing wound while these tapes are in place.

In circumstances in which the sutures, tapes or staple materials are dissolvable *in situ,* the material releases the selected therapeutic agent as the material dissolves and can deliver doses of the agents over time, *e*.*g*., 1 day to about 14 days. In circumstances in which the wound closure materials do not dissolve, *e*.*g*., staples, the agents are still delivered which the wound is in contact with the staple or tape.

Various methods of association may be selected depending upon the nature of the agent (small molecule, protein, DNA/RNA) and the nature of the wound closure material. For instance, the physical association can include coating a liquid solution containing the agent onto the wound closure material, such as sutures, staples or tapes, and allowing the solution to dry.

Alternatively, the agent may be chemically associated with the material by means of a covalent or non-covalent bond or cross-link formed between the agent and the wound closure material. One or more agents may be are covalently linked or cross-linked directly to a chemical side group of the wound closure material. In one disclosure the agents are linked to a catgut suture having multiple amino acid side chains to which small molecule or peptide agents are attached via covalent bonds. For example, one such composition is a catgut suture having multiple amino acid side chains to which 1,4-DPCA molecules are attached via covalent bonds. In one disclosure, one or more agents are attached to the wound closure material via a crosslinker, such as the imidoester crosslinker dimethyl suberimidate, the N-Hydroxysuccinimide-ester crosslinker BS3, formaldehyde, zero-length carbodiimide crosslinker EDC (Carbodiimide), DCC (dicyclohexyl carbodiimide), glutaraldehyde (GA), beta-cyclodextrin polyrotaxane monoaldehyde, SMCC or its water-soluble analog, Sulfo-SMCC, a NHS ester compound, a Maleimide compound, a Hydrazide compound, an imidoester, an Aldehyde, 2,5-dimethoxy-2,5-dihydro-furan (DMDF)-iodine, 2, 5-Dimethoxy-2, 5-Dihydro-Furan (DMDF), Genipin or a Pyridyldithiol reagent. See, e.g., Thermo Scientific - Crosslinking Technical Handbook, retrieved from tools.thermofisher.com/content/sfs/brochures/1602163-Crosslinking-Reagents-Handbook.pdf; CAMENZIND et al., Can Genipin-coated Sutures Deliver a Collagen Crosslinking Agent to Improve Suture Pullout in Degenerated Tendon? An Ex Vivo Animal Study. Clin Orthop Relat Res. 2018 May;476(5):1104-1113; ZHAO et al, Collagen membranes crosslinked by β-cyclodextrin polyrotaxane monoaldehyde with good biocompatibilities and repair capabilities for cornea repair. RSC Advances, Issue 46, 2017, Issue in Progress; and FRANCIS et al., 2, 5-Dimethoxy-2, 5-Dihydro-Furan (DMDF) crosslinked radio-opaque biodegradable antimicrobial sutures. Front. Bioeng. Biotechnol. Conference Abstract: 10th World Biomaterials Congress. doi: 10.3389/conf.FBIOE.2016.01.00787.

Additionally, one or more agents may be covalently linked to the wound closure material via a linker. The linker can itself be a small molecule, a chemical compound or series thereof, a polymer, one or more nucleic acids, or one or more amino acids, depending upon the nature of the association desired between the materials. In one disclosure, the polymer linker is a polyethylene glycol. In another disclosure, the polymer linker is a polylactic acid. In yet another disclosure, the polymer linker is a polyglycolic acid.

Another disclosure involves generating the wound closure/wound healing material such that the agent is infused into the material during generation of the material. As one disclosure, in which the material is a PLA/GPLA suture mixture, a small molecule or peptide agent is infused into the polymer mixture during generation of the material, resulting in a suture containing embedded agent. For example, when the material is a PLA/GPLA suture mixture, 1,4-DPCA molecules are infused into the mixture during generation of the material resulting in a wound closure suture that releases 1,4-DPCA during healing and during the process of the suture dissolving *in situ.* Sutures spooled from glycolic and lactic acid mixtures (*e*.*g*. Monocryl) also degrade fully after 2 weeks. The addition of 1,4-DPCA to the spooled mixture engender a nearly scarless healing suture. Without wishing to be bound by theory, the inventors believe that coupling the drug enables a slow release system over about 2 weeks. Incorporation of 1,4-DPCA is particularly important since lactic acid gets released from the polymer and can then promote a metabolic shift from oxidative phosphorylation to aerobic glycolysis, which in turn would complement the drug/reagent's activity that encourages the same metabolic shift. In summary, the reagent plus the lactic acid would enable an enhanced anti-scarring, pro-regenerative response.

As disclosed herein, the wound closure materials may be generated in association with selected agents to create the composition. In a further disclosure, 1,4-DPCA or related compounds/reagents are incorporated into other wound closure materials used with or instead of sutures As above, the same coupling reactions produce a material that exists as a fibrous structure or other formulations used to cover wounds (such as a tape or adhesive), or other materials to promote regenerative healing where needed or desired. Such materials could provide slowly released drug directed at the skin, the subdermal layers, and the tissue beneath it, encouraging an epimorphic regenerative response.

Therefore, also provided herein is a method for reducing scarring or improving skin integrity during healing of a mammalian wound comprising covering or closing a wound with a wound-closure material physically or chemically associated with an agent that reduces scarring and improves the integrity of skin and underlying tissue as described herein. One disclosure of the method involves suturing a mammalian wound with a suture impregnated, coated or chemically linked to an agent such as a PHD inhibitor or other small molecule, peptide or DNA/RNA sequence described herein. Another disclosure of the method involves stapling the edges of a mammalian wound with a staple, coated or chemically linked to an agent such as a PHD inhibitor or other small molecule, peptide or DNA/RNA sequence described herein. Another disclosure of the method involves closing a mammalian wound with an adhesive tape coated with, chemically linked to, or impregnated with an agent such as a PHD inhibitor or other small molecule, peptide or DNA/RNA sequence described herein. Each method permits the selected therapeutic agent to be delivered in effective dosages to the healing wound, thus reducing scarring and regenerating healthy tissue.

The compositions and methods described herein may allow for delivery of the agent over a similar 2-week period while the composition dissolves or before the composition is removed, thereby releasing the compound drug over an effective period of time. Since sutures, staples, and wound binding tapes interact directly with the skin, the inventors theorize that these modified sutures, staples, and adhesives lead to a nonscarring heal (regenerative) response, which is ideal for treatment of facial wounds, plastic surgery, as well as skin anywhere on the body.

The compositions and methods described herein are anticipated (a) to reduce incidence, severity, or both, of the cutaneous scar without impairing normal wound healing and (b) to treat the cutaneous scar in the subject, such that at least one of the wound size, scar area, and collagen whorl formation in the wound is reduced compared to a control.

### Examples

The following examples disclose wound closure compositions and their uses in reducing scarring.

### EXAMPLE 1: MODIFIED SUTURES

A coated suture-based delivery system of the regenerative compound 1,4-DPCA is created using a coating similar to that described by LI, Y et. al., New Bactericidal Surgical Suture Coating, Langmuir, 2012 Aug; 28(33):12134-12139. This coating consists of a poly(lactic-co-glycolic acid) backbone with protruding aminoethyl methylacrylate and butyl methylacrylate groups along the length of the backbone. In the synthetic scheme of LI *et al,* cited above, we replace the coupled aminoethyl methylacrylate and butyl methylacrylate groups with 1,4-DPCA in order to generate a coated, wound-healing, drug-eluting suture. We activated the carboxylic esters of the side chains with 1,1'-carbonyldiimidazole followed by an addition of 1,4-DPCA. The carboxylate of the 1,4-DPCA displaces the imidazole from the activated suture coating via a simple nucleophilic substitution.

For cat gut sutures (*e*.*g*. used in facial surgery), multiple amino acid side chains on the suture material are suitable for chemical coupling of the compound, and as the suture degrades, the drug is released. Cat gut is made of extracellular matrix components with the majority being collagen. The more collagen, the better the suture. Since 1,4-DPCA inhibits prolyl hydroxlases that crosslink collagen in the extracellular matrix, which promotes scarring, addition of this compound to the gut suture is anticipated to reduce scar formation two ways, by promoting epimorphic regeneration and by reducing crosslinking of the wound-induced extracellular matrix.

### EXAMPLE 2: INCORPORATING 1,4-DPCA IN A MIXTURE FOR FORMULATION WITH PLA TO GENERATE SUTURES

For polymer sutures spooled from glycolic mixtures, such as polylactic acid mixtures (*e*.*g*. Monocryl), the addition of 1,4-DPCA (or an analogous reagent as described herein) to the chemical mixture spooled into the polymer suture creates a scarless healing suture. For this application, the compound is simply captured in polymer rather than chemically coupled. For example, PLA (0.1 gm) is dissolved in CH₃Cl (1 ml) and 100 µg DPCA. The solution is mixed well and dried, and then suture is spooled therefrom.

Where lactic acid is released from a polylactic acid-based polymer suture, this event reinforces a metabolic shift in sutured tissue from oxidative phosphorylation (OxPhos) to aerobic glycolysis, reinforcing epimorphic healing which stimulates the same metabolic shift. Sutures that degrade over 2-3 weeks offer a perfect time release period for 1,4-DPCA.

### EXAMPLE 3: IN VITRO DRUG RELEASE FROM SUTURE

A PLA/PGLA suture, infused with 1,4-DPCA molecules during generation of the sutures as described in Example 1 (i.e., materials are melted, DPCA added, and the final mixture cooled and pulled into sutures), was tested for rate of release of 1,4-DPCA into DMEM medium. Drug release was measured by HIF levels. The sutures were soaked in medium without serum for 24 hours and transferred to new medium for each timepoint (1 hour, 3 hours, Days 1, 2, 3, 6, 7, 8, 9, 10, 13, 14, 15, 16, 17 and 20). The medium was tested for HIP1a protein activity in a luciferase assay (Signosis, Inc) as described in ESKLA, K-L et al, Hypothermia augments stress response in mammalian cells. Free Radical Biology and Medicine, 2018 doi:10.1016/j.freeradbiomed.2018.04.571.

The results are shown in the graph at FIG. 1. The two heavy lines represented PLA/PLGA[50:50] 50:50 +DPCA and PLA/PLGA[50:50] 75:25 +DPCA are shown releasing the drug after one hour in the medium up to about 15 days in the medium after which the drug detection falls under the DMEM control.

### EXAMPLE 4: IN VIVO DRUG RELEASE FROM SUTURE

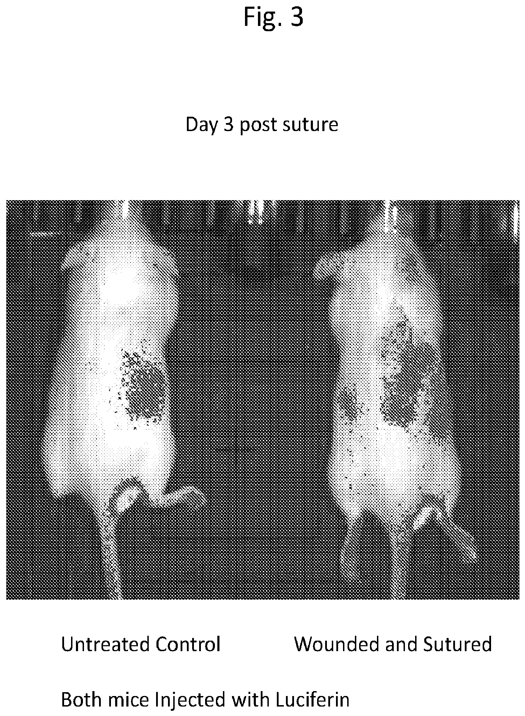

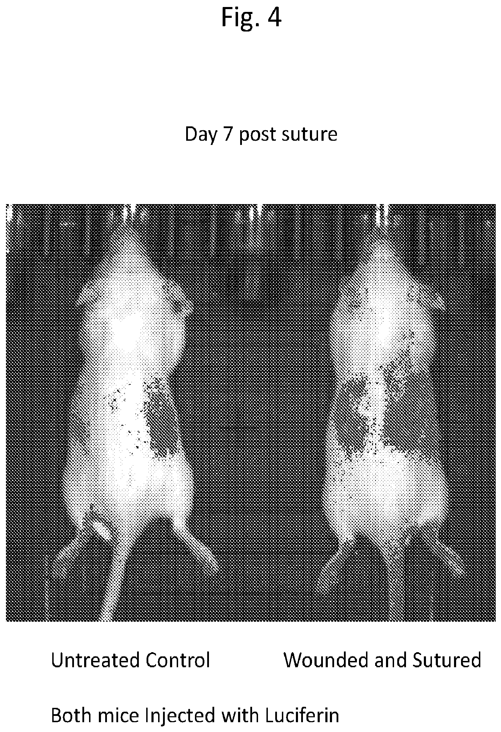

One HIF reporter mouse was injected with luciferin but otherwise untreated as a control and a second HIF reporter mouse was given a 1 cm linear incision its right side. The incision was sutured with a suture as described in Example 2 sandwiched between two silk sutures. In the presence of luciferin, the HIF reporter mouse demonstrates bioluminescence where HIF is produced. The bioluminescence of both mice were observed at various times post-injury. The increase in bioluminescence at Day 1 post-suture indicates that the drug was being detected at the point of the suture, as evidenced by an increase in HIF detected by luciferin (FIG. 2). At Day 3 post suture, additional bioluminescence is observed at the site of the wound (FIG. 3). At Day 7 post suture, still more bioluminescence is observed at the site of the wound. Finally at Day 15 post-suture (FIG. 4), the bioluminescence is reduced. These results demonstrate that release of the drug from the suture *in vivo* is similar to release *in vitro* in Example 3.

### EXAMPLE 5: OBSERVATION OF EPIDERMIS AFTER APPLICATION OF SUTURES

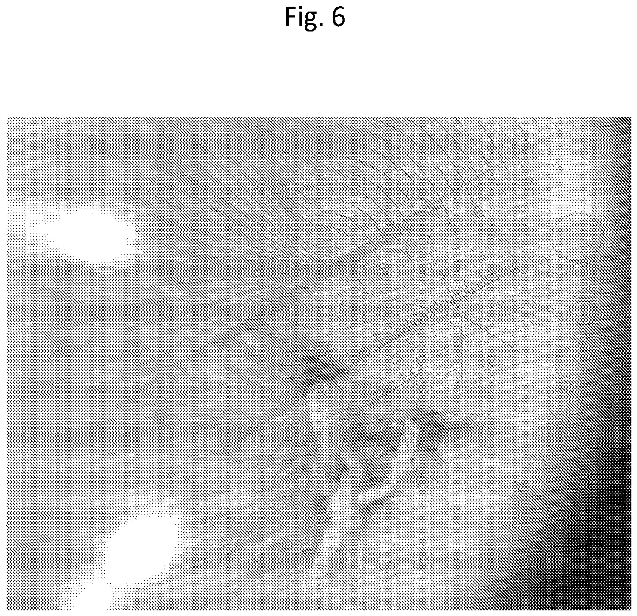

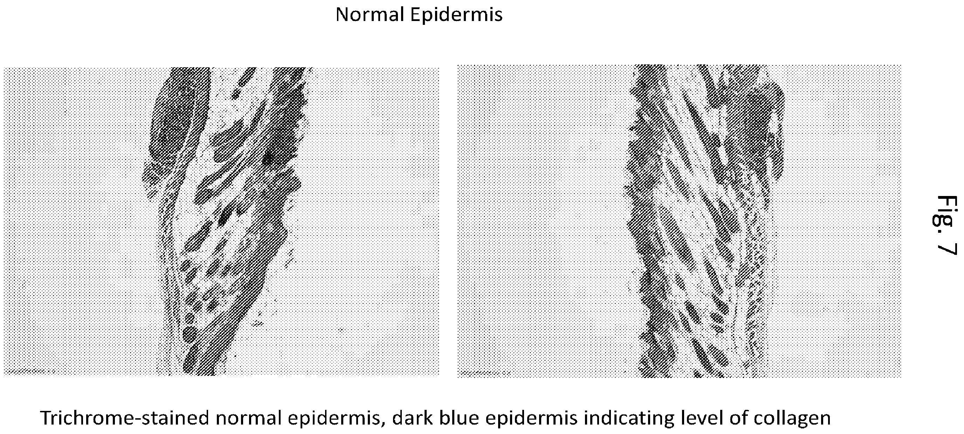

An HIF reporter mouse is given 1 cm linear incision on the skin (epidermis) of the right side of mouse skin. The incision was sutured with a suture of Example 2, sandwiched between two silk sutures. FIG 6 depicts the initial suture. For comparison pictures of normal epidermis of a HIF reporter mouse, that was trichrome stained and photomicrographs were taken. As seen in FIG. 7, the darker (blue) epidermis indicates the level of collagen.

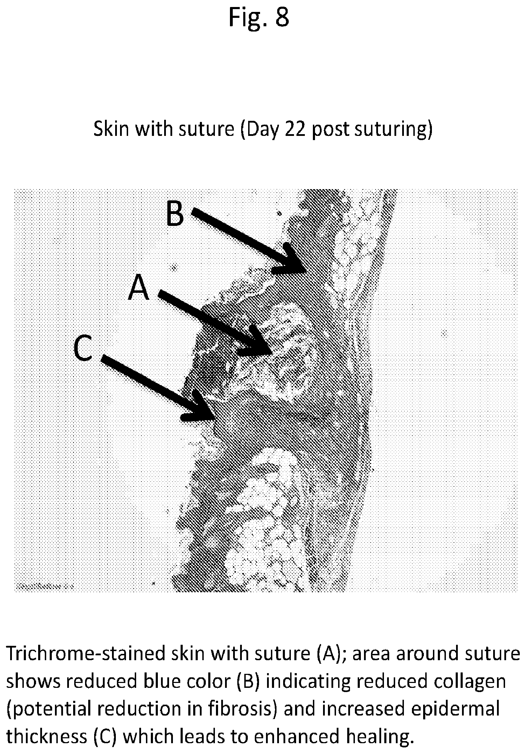

Twenty-two days after the initial incision and suturing, a histological analysis of the wound area around the treated suture was made. The trichrome-stained skin of the mouse that the area around the suture has a reduced blue color (see FIG. 8, point B), indicating reduced collagen (i.e., a potential reduction in fibrosis). The skin of the mouse also demonstrates increased epidermal thickness (see FIG. 8 at point C). These two physiological observations in the skin lead to enhanced healing.

## Claims

1. A composition comprising a suture material chemically associated with an agent that reduces scarring and improves the integrity of skin and underlying tissue in a mammalian subject, wherein the agent is a small molecule prolyl hydroxylase inhibitor (PHDi) compound,
wherein the PHDi is 1,4-dihydrophenonthrolin-4-one-3-carboxylic acid (1,4-DPCA) or Fibrogen (FG) 4592, and
wherein the suture is associated with the agent via a covalent or non-covalent bond or cross-link formed between the agent and the suture, or infusion of the agent into the suture material during generation of the material.

2. The composition according to claim 1, wherein the suture is a catgut protein suture or a suture spooled from mixtures of glycolic and lactic acid or silk.

3. The composition according to claim 2, wherein the wound closure material is dissolvable and wherein the material releases the agent as the material dissolves in situ.

4. The composition according to claim 1, wherein the agent is covalently linked or cross-linked directly to a chemical side group of the wound closure material, optionally via a linker.

5. The composition according to claim 4, wherein the linker is a polymer, optionally a polyethylene glycol, a polylactic acid, or polyglycolic acid.

6. The composition according to claim 1, wherein the wound closure material is a catgut suture having multiple amino acid side chains to which small molecule prolyl hydroxylase inhibitor agents are attached via covalent bonds.

7. The composition according to claim 6, wherein the wound closure material is a catgut suture having multiple amino acid side chains to which 1,4, DPCA molecules are attached via covalent bonds.

8. The composition according to claim 1, wherein the material is a PLA/GPLA suture mixture into which a small molecule prolyl hydroxylase inhibitor agent is infused during generation of the material.

9. The composition according to claim 8, wherein the material is a PLA/GPLA suture mixture into which 1,4, DPCA molecules are infused during generation of the material.

10. The composition of claims 1 to 9 for use in the reduction of scarring or improvement of skin integrity during the healing of a mammalian wound.

11. The composition for use according to claim 10, wherein the wound is sutured with a suture impregnated, coated or chemically linked to an agent that reduces scarring and improves the integrity of skin and underlying tissue.
